# EUROPEAN PATENT APPLICATION

(11) **EP 2 153 827 A2**
(43) Date of publication of application: **17.02.2010**
(21) Application number: 09176388.8
(22) Date of filing: 27.09.2001
(51) Int. Cl.: A61K 9/70, A61K 31/4468

(54) **Composition for the Transdermal Delivery of Fentanyl**

(30) Priority: 29.09.2000 US 236973 P; 16.04.2001 US 284017 P
(62) Divisional of application: 01983925.7
(71) Applicant: 3M Innovative Properties Company, St. Paul MN 55133-3427 (US)
(72) Inventor: Cantor, S. Adam, River Falls, WI 54022 (US); Ocheltree, W. Terrance, Lexington, KY 40509 (US); Robles, A. Cynthia, New Richmond, WI 54017 (US)
(74) Representative: Vossius & Partner

(57) **Abstract**

The present invention relates to a device for the transdermal delivery of fentanyl comprising a backing and a transdermal drug delivery composition, said transdermal drug delivery composition being adhered to one surface of the backing, wherein said transdermal drug delivery composition comprises
(a) a copolymer comprising
(i) one or more A monomers selected from the group consisting of alkyl acrylates containing 4 to 12 carbon atoms in the alkyl group and alkyl methacrylates containing 4 to 12 carbon atoms in the alkyl group; and
(ii) one or more ethylenically unsaturated B monomers copolymerizable with the A monomer; and

(b) about 8% to about 30% by weight fentanyl based on the total weight of the composition.

## Description

This application claims priority to U.S. Provisional Patent Application No. 60/236,973, filed September 29, 2000 and to U.S. Provisional Patent Application No. 60/284,017, filed April 16, 2001.

### Field of the Invention

The present invention relates to a transdermal drug delivery composition containing fentanyl. The invention further relates to a transdermal drug delivery device for the delivery of fentanyl and to methods of providing sustained analgesia to subjects in need thereof.

### Background of the Invention

Transdermal drug delivery devices are designed to deliver a therapeutically effective amount of drug across the skin of a patient. Transdermal drug delivery devices typically involve a carrier (such as a liquid, gel, or solid matrix, or a pressure sensitive adhesive) into which the drug to be delivered is incorporated. Devices known to the art include reservoir type devices involving membranes that control the rate of drug release to the skin and devices where the drug is dispersed or dissolved in a matrix such as a pressure sensitive adhesive.

It has long been known that fentanyl is an extremely potent and effective anesthetic and analgesic. Fentanyl is most frequently administered as the citrate salt intravenously (IV) or intramuscularly (IM) to achieve therapeutic effects. Fentanyl citrate is preferred for injection because of its aqueous solubility. Fentanyl may also be administered as a transdermal patch or as a lozenge. Additional details regarding pharmacokinetics, uses, and dosages of fentanyl may be found in the monograph "Fentanyl Citrate", AHFS 98 Drug Information, ed.: G. K. McEvoy, American Society of Health-Systems Pharmacists, p.1677-1683 (1998).

Following IV or IM administration the onset of action is very rapid but the decrease in serum fentanyl concentration is also rapid, which necessitates dosing at frequent intervals. Following IV administration the onset of action is within a few minutes with a 30 to 60 minute duration. Following intramuscular administration the onset of action is about 10 minutes with a 1 to 2 hour duration. Minimum effective analgesic serum levels of fentanyl range from 0.2 to 2 ng/mL.

Oral absorption is low presumably due to a high hepatic clearance by first-pass metabolism. Lozenges that provide a combination of transmucosal and oral dosage are indicated for treatment of breakthrough cancer pain, but also have a short duration of action.

Transdermal administration of fentanyl can overcome the drawbacks of frequent dosing needed with the aforementioned routes of administration. This can also avoid the peaks and valleys obtained with pulsatile delivery, making it easier to maintain therapeutic doses without causing serious side effects that may result from peak serum levels.

A fentanyl transdermal system described in U. S. Patent No. 4,588,580 that provides continuous systemic delivery of fentanyl for 72 hours is available under the tradename Duragesic®.

With regard to a specific transdermal device there are a number of properties that the device should optimally include, and design of an effective transdermal drug delivery device often involves finding a suitable balance among these properties, since they can oftentimes be mutually exclusive.

The device needs to provide sufficient skin flux of the active compound so that it does not need to be excessively large in size, but it should also control the rate of delivery sufficiently to avoid an overdosing effect.

The device needs to contain an adequate amount of the active compound so that it does not become depleted before the end of the designated dosage period. The dosage period is typically 1 to 7 days.

The device should be designed to make it difficult to accidentally deliver higher dosages than the intended amount (i.e., avoid dose dumping).

The device needs to remain stable both with regards to the chemical stability of the active compound and with regards to the physical stability of the device itself, so that it continues to perform as intended after aging.

The device should be non-irritating to skin with regards to chemical sensitivity, chemical irritation, and mechanical irritation, since it is affixed to an external part of the body for extended periods of time.

The device should be attractive or unobtrusive for the patient, or otherwise have visual characteristics that assist with the therapy.

The device should be easy to manufacture, and will optimally have a fairly simple design.

### Summary of the Invention

The present invention provides compositions for the transdermal delivery of fentanyl. In one aspect of the invention the composition comprises:
(a) a copolymer comprising
   (i) one or more A monomers selected from the group consisting of alkyl acrylates containing 4 to 12 carbon atoms in the alkyl group and alkyl methacrylates containing 4 to 12 carbon atoms in the alkyl group; and
   (ii) one or more ethylenically unsaturated B monomers copolymerizable with the A monomer; and
(b) about 8% to about 30% by weight fentanyl.

The present invention also provides a composition for the transdermal delivery of fentanyl comprising:
(a) a pressure sensitive adhesive;
(b) fentanyl; and
(c) a delivery enhancing adjuvant selected from the group consisting of methyl laurate, tetraglycol, and mixtures thereof.

The present invention still further provides a method of providing sustained analgesia to a mammal comprising delivering fentanyl to a mammal via a transdermal drug delivery device in an amount of about 0.5 to about 5.0 mg/day thereby causing the serum concentration of fentanyl in the mammal to be about 0.2 to about 10 ng/mL for a period of time from about 4 to about 14 days.

The compositions of the present invention may be adhered to one surface of a backing to create a transdermal drug delivery device.

The transdermal drug delivery device of the present invention is useful to induce analgesia.

### Detailed Description of the Invention

The invention provides compositions for the transdermal delivery of fentanyl and transdermal drug delivery devices containing these compositions.

One transdermal drug delivery composition of the present invention comprises a copolymer of alkyl (meth)acrylate A monomers in which the alkyl group has 4 to 12 carbon atoms and ethylenically unsaturated B monomers that are copolymerizable therewith.

Suitable acrylate copolymers for use in the composition preferably comprise about 40 to about 95 percent by weight, more preferably about 50 to about 70 percent by weight, based on the total weight of all monomers in the copolymer, of one or more A monomers selected from the group consisting of alkyl acrylates containing 4 to 12 carbon atoms in the alkyl group and alkyl methacrylates containing 4 to 12 carbon atoms in the alkyl group. Examples of suitable alkyl acrylates and methacrylates include n-butyl, n-pentyl, n-hexyl, isoheptyl, n-nonyl, n-decyl, isohexyl, 2-ethyloctyl, isooctyl and 2-ethylhexyl acrylates and methacrylates. Preferred alkyl acrylates include isooctyl acrylate, 2-ethylhexyl acrylate, n-butyl acrylate, and cyclohexyl acrylate. Isooctyl acrylate is a particularly preferred A monomer.

The acrylate copolymer further comprises about 5 to about 55 percent by weight, more preferably about 5 to about 45 percent by weight, based on the total weight of all monomers in the copolymer, of one or more B monomers. Suitable B monomers include those containing a functional group selected from the group consisting of carboxylic acid, sulfonamide, urea, carbamate, carboxamide, hydroxy, amino, oxy, oxo, and cyano. Exemplary B monomers include acrylic acid, methacrylic acid, maleic acid, a hydroxyalkyl acrylate containing 2 to 4 carbon atoms in the hydroxyalkyl group, a hydroxyalkyl methacrylate containing 2 to 4 carbon atoms in the hydroxyalkyl group, acrylamide, methacrylamide, an alkyl substituted acrylamide containing 1 to 8 carbon atoms in the alkyl group, N-vinyl-N-methyl acetamide, N-vinyl valerolactam, N-vinyl caprolactam, N-vinyl-2-pyrrolidone, glycidyl methacrylate, vinyl acetate, alkoxyethyl acrylate containing 1 to 4 carbon atoms in the alkoxy group, alkoxyethyl methacrylate containing 1 to 4 carbon atoms in the alkoxy group, 2-ethoxyethoxyethyl acrylate, furfuryl acrylate, furfuryl methacrylate, tetrahydrofurfuryl acrylate, tetrahydrofurfuryl methacrylate, propylene glycol monomethacrylate, propylene oxide methyl ether acrylate, di(lower)alkylamino ethyl acrylate, di(lower)alkylamino ethyl methacrylate, di(lower alkyl)aminopropyl methacrylamide, acrylonitrile, and methacrylonitrile. Preferred B monomers include 2-hydroxyethyl acrylate, acrylamide, and vinyl acetate. A particularly preferred B monomer is 2-hydroxyethyl acrylate.

The copolymer may optionally further comprise a substantially linear macromonomer copolymerizable with the A and B monomers and having a weight average molecular weight in the range of about 500 to about 500,000, preferably about 2,000 to about 100,000 and more preferably about 4,000 to about 20,000. The macromonomer, when used, is generally present in an amount of not more than about 20% and preferably not more than about 10% by weight based on the total weight of all monomers in the copolymer. Suitable macromonomers include functionally terminated polymethylmethacrylate, styrene/acrylonitrile, polyether, and polystyrene macromonomers. Examples of useful macromonomers and their preparation are described in Krampe et al., U.S. Patent No. 4,693,776, the disclosure of which is incorporated herein by reference. Polymethylmethacrylate macromonomers are particularly preferred.

The copolymers described above can be prepared by methods well known to those skilled in the art and described for example in U.S. Patent No. RE 24,906 (Ulrich), U.S. Patent No. 4,732,808 (Krampe), and International Publication Number WO 96/08229 (Garbe), the disclosures of which are incorporated herein by reference.

The inherent viscosity of the copolymer is such as to ultimately provide a suitable pressure sensitive adhesive when used in a composition or device of the invention. Preferably the copolymer has an inherent viscosity in the range of about 0.2 dL/g to about 2.0 dL/g, more preferably about 0.3 dL/g to about 1.4 dL/g.

Fentanyl is present in the composition in an amount between about 8% and about 30% by weight, preferably between about 12% and 24% by weight, based on the total weight of the composition. In a preferred embodiment, the composition is substantially free or free of undissolved fentanyl. The presence of undissolved fentanyl may be detected by examination with an optical microscope at 20x magnification. The ability to dissolve high concentrations of fentanyl in these compositions provides a number of benefits, including the ability to deliver therapeutic amounts of fentanyl for extended periods of time, for example about 4 to about 14 days and preferably about 7 days. The particular amount of fentanyl in the composition that will deliver sufficient fentanyl to achieve a desired therapeutic result varies according to the condition being treated, any drugs being coadministered with the fentanyl, desired duration of treatment, the surface area and location of the skin over which the device is to be placed, and the selection of adjuvant and other components of the transdermal delivery device. If desired, the composition can contain components that modify the properties of the copolymer, such as plasticizers, tackifiers, and the like of types and in amounts readily determinable to those of skill in the art.

In a preferred embodiment the composition of the present invention further comprises an adjuvant that enhances the transdermal delivery of fentanyl. Any adjuvant that enhances the transdermal delivery of fentanyl may be used in the composition of the invention regardless of the way in which such enhancement is achieved.

Suitable adjuvants include certain pharmaceutically acceptable materials that have been used as skin permeation enhancers or solubilizers in transdermal drug delivery systems. Exemplary materials include C₈-C₃₆ fatty acids such as isostearic acid, octanoic acid, and oleic acid; C₈-C₃₆ fatty alcohols such as oleyl alcohol and lauryl alcohol; lower alkyl esters of C₈-C₃₆ fatty acids such as ethyl oleate, isopropyl myristate, butyl stearate, and methyl laurate; di(lower) alkyl esters of C₆-C₈ diacids such as diisopropyl adipate; monoglycerides of C₈-C₃₆ fatty acids such as glyceryl monolaurate; tetraglycol (tetrahydrofurfuryl alcohol polyethylene glycol ether); tetraethylene glycol (ethanol,2,2'-(oxybis(ethylenoxy))diglycol); C₆-C₃₆ alkyl pyrrolidone carboxylates; polyethylene glycol; propylene glycol; 2-(2-ethoxyethoxy)ethanol; diethylene glycol monomethyl ether; N,N-dimethyldodecylamine N-oxide; and combinations of the foregoing. Alkylaryl ethers of polyethylene oxide, polyethylene oxide monomethyl ethers, and polyethylene oxide dimethyl ethers are also suitable, as are solubilizers such as glycerol and N-methyl pyrrolidone. The terpenes are another useful class of softeners, including pinene, d-limonene, carene, terpineol, terpinen-4-ol, carveol, carvone, pulegone, piperitone, menthone, menthol, neomenthol, thymol, camphor, borneol, citral, ionone, and cineole, alone or in any combination.

Preferred delivery enhancing adjuvants include ethyl oleate, isopropyl myristate, glycerol, tetraglycol, methyl laurate, N, N-dimethyldodecylamine N-oxide, limonene, terpineol, tetraethylene glycol, propylene glycol, and menthol. Particularly preferred delivery enhancing adjuvants are tetraglycol and methyl laurate.

In a composition of the invention the adjuvant(s) is dispersed, preferably substantially uniformly, and more preferably dissolved in the composition and is present in an amount that enhances fentanyl permeation through the skin compared to a like composition not containing the adjuvant(s) when this phenomenon is measured using the skin permeation model described below. The total amount of delivery enhancing adjuvant will generally be about 5 to about 40% by weight based on the total weight of the composition.

The physical properties desirable in a transdermal drug delivery device are well known to those skilled in the art. For example, it is desirable to have sufficiently little cold flow that a device of the invention is stable to flow upon storage. It is also preferred that it adhere well to the skin and release cleanly from the skin. In order to achieve resistance to cold flow, preferred levels of skin adhesion and clean release, the amount and structure of the comonomers in the copolymer, the inherent viscosity of the copolymer, and the amount and type of adjuvant are selected such that the adhesive layer(s) obtain the desired balance of these properties.

The invention additionally provides a pressure sensitive adhesive composition for the transdermal delivery of fentanyl comprising a polymer, fentanyl, and a delivery enhancing adjuvant selected from the group consisting of methyl laurate, tetraglycol, and mixtures thereof.

Examples of suitable types of polymers for use in the pressure sensitive adhesive composition include acrylates, natural and synthetic rubbers such as polyisobutylenes, polysiloxanes, polyurethanes, and other polymers known in the art to be useful as components of pressure sensitive skin adhesive compositions. The polymers can be present alone or in combination. The acrylate copolymers described in detail above are preferred pressure sensitive adhesives for use in the compositions of the invention.

In this embodiment of the invention fentanyl is present in the composition in an amount between about 1% and about 30% by weight, preferably between about 5% and about 24% by weight, based on the total weight of the composition. Preferably the composition is substantially free or free of undissolved fentanyl. The presence of undissolved fentanyl may be detected by examination with an optical microscope at 20x magnification. The particular amount of fentanyl in the composition that will deliver sufficient fentanyl to achieve a desired therapeutic result varies according to the condition being treated, any drugs being coadministered with the fentanyl, desired duration of treatment, the surface area and location of the skin over which the device is to be placed, and the selection of adjuvant and other components of the transdermal delivery device. If desired, the composition can contain components that modify the properties of the copolymer, such as plasticizers, tackifiers, and the like of types and in amounts readily determinable to those of skill in the art.

The total amount of delivery enhancing adjuvant will generally be about 5 to about 40% by weight based on the total weight of the composition. The invention further provides a method of providing sustained analgesia to a mammal comprising delivering fentanyl to a mammal via a transdermal drug delivery device in an amount of about 0.5 to about 5.0 mg/day thereby causing the serum concentration of fentanyl in the mammal to be about 0.2 to about 10 ng/mL for a period of time from about 4 to about 14 days. In a preferred embodiment, the device provides transdermal administration to a mammal of 0.5 to 2.5 mg/day of fentanyl thereby causing the serum concentration of fentanyl in the mammal to be 0.3 to 4 ng/mL for a period of about 6 to about 8 days. Preferred transdermal drug delivery devices contain the compositions described above for the transdermal delivery of fentanyl.

The amount of fentanyl that needs to be delivered and the serum concentrations that are necessary to be therapeutically effective show considerable variation between individuals. A tolerance to fentanyl generally develops with continued use, typically necessitating the need for increased dosages over time of treatment. Because of this inter- and intra- patient variation, a wide range of therapeutically effective fentanyl serum concentrations have been reported. Further details may be found in the monographs "Fentanyl Citrate", AHFS 98 Drug Information, ed.: G. K. McEvoy, American Society of Health-Systems Pharmacists, p.1677-1683 (1998) and "Fentanyl: A Review for Clinical and Analytical Toxicologists", A. Poklis, Clinical Toxicology, 33(5), 439-447 (1995).

A transdermal delivery device of the invention also comprises a backing. The backing is flexible such that the device conforms to the skin. Suitable backing materials include conventional flexible backing materials used for pressure sensitive adhesive tapes, such as polyethylene, particularly low density polyethylene, linear low density polyethylene, metallocene polyethylenes, high density polyethylene, polypropylene, polyesters such as polyethylene terephthalate, randomly oriented nylon fibers, ethylenevinyl acetate copolymer, polyurethane, natural fibers such as rayon and the like. Backings that are layered such as polyethylene terephthalate-aluminum-polyethylene composites are also suitable. The backing should be substantially inert to the components of the adhesive layer.

Transdermal devices of the invention are preferably prepared by combining the copolymer, the adjuvant and the fentanyl with an organic solvent (e.g., ethyl acetate, isopropanol, methanol, acetone, 2-butanone, ethanol, toluene, alkanes, and mixtures thereof) to provide a coating composition. The mixture is shaken or stirred until a homogeneous coating composition is obtained. The resulting composition is then applied to a release liner using conventional coating methods (e.g., knife coating or extrusion die coating) to provide a predetermined uniform thickness of coating composition. Suitable release liners include conventional release liners comprising a known sheet material such as a polyester web, a polyethylene web, a polystyrene web, or a polyethylene-coated paper coated with a suitable fluoropolymer or silicone based coating. The release liner that has been coated with the composition is then dried and laminated onto a backing using conventional methods.

The transdermal delivery devices of the invention can be made in the form of an article such as a tape, a patch, a sheet, a dressing or any other form known to those skilled in the art. Generally, the device will be in the form of a patch of a size suitable to deliver a preselected amount of fentanyl through the skin. Generally, the device will have a surface area of about 5 cm² to about 100 cm² and preferably about 10 cm² to about 40 cm².

Another preferred transdermal drug delivery device of the invention contains at least three distinct layers in addition to a backing layer. The first layer is adhered to the backing and comprises a transdermal drug delivery composition of the present invention that serves as a drug reservoir. The second layer comprises a rate controlling membrane that is adhered to the surface of the first layer opposed to the surface-in contact with the backing. The third layer comprises a pressure sensitive adhesive that is adhered to the surface of the membrane that is opposed to the surface of the membrane in contact with the first layer. This third layer contacts the skin of the subject when the device is used (the "skin contacting layer"). This type of device is referred to as a "membrane rate controlled device".

The membrane is selected such that it is rate controlling, i.e., the presence of the membrane in the device may change the skin penetration profile of the device compared to a like device not having the membrane. Suitable membranes include continuous film membranes and microporous membranes. Preferred membranes are continuous film membranes prepared from ethylene:vinyl acetate copolymers containing from about 0.5 to about 28 wt-% vinyl acetate. Most preferred membranes are continuous film membranes prepared from ethylene:vinyl acetate copolymers containing about 2 to about 9 wt-% vinyl acetate. The membrane thickness will generally be from about 25 µm to about 100 µm, preferably the thickness will be about 50 µm.

Because the delivery rate of the drug is controlled by the membrane, a variety of pressure sensitive adhesives that have a range of affinities for the drug may be used in the third (skin contacting) layer. The pressure sensitive adhesive used in the skin contracting layer can be the same as or different from the transdermal drug delivery composition used in the reservoir layer. Pressure sensitive adhesives used in the skin contacting layer preferably comprise polymers selected from the group consisting of acrylates, natural rubbers, synthetic rubbers such as polyisobutylenes, polyisoprenes, styrene block copolymers and silicone polymers. Particularly preferred is to have the pressure sensitive adhesive used in the skin contacting layer be the same as the transdermal drug delivery composition used in the reservoir layer.

The skin contacting layer can initially contain drug in a concentration similar to that of the reservoir layer or the skin contacting layer can contain no drug, since it is expected that over time drug will diffuse from the reservoir layer into the skin contacting layer.

In another embodiment, the transdermal drug delivery device of the invention contains at least two distinct layers in addition to a backing layer. The first layer, also known as the reservoir, is adhered to the backing and comprises a transdermal drug delivery composition of the present invention that serves as a drug reservoir. The second layer, also known as the "rate controlling layer", comprises a pressure sensitive adhesive layer that is adhered to the surface of the first layer opposed to the surface in contact with the backing. The rate controlling layer contacts the skin of the subject. The rate controlling layer serves to control the rate of delivery of the drug to the subject and to adhere the device to the subject's skin. Thus the presence of the rate controlling layer in the device may change the skin penetration profile of the device compared to a like device where the rate controlling layer is identical in composition to the reservoir layer. This control of rate of delivery of the drug may be due to differences in the affinity of the drug for the two different layers and differences in the rate of diffusion of the drug through the two different layers. These differences in affinity and/or diffusion of the drug in the two layers, as well as the relative thickness of the layers, allows the rate of delivery of the drug to be controlled. This system is referred to as an "adhesive rate controlled system".

In a preferred embodiment of the adhesive rate controlled system, the two layers are selected so that the second (rate controlling) layer has a lower affinity for the drug than the first (reservoir) layer. By "lower affinity" is meant that the drug preferentially resides in the reservoir layer, so that when the system is at equilibrium the weight percentage of drug in the reservoir layer is greater than the weight percentage of drug in the rate controlling layer. The difference in the affinity of the two layers for the drug, as well as the relative thickness of the layers, allows the rate of delivery of the drug to be controlled.

The rate controlling layer differs in composition from the reservoir layer. The first and second layers may contain, for example, different types and amounts of polymers, including polymers that differ in their extent of reaction, crosslinking, branching, and copolymer sequences. Pressure sensitive adhesives of the rate controlling layer preferably comprise polymers selected from the group consisting of acrylates, natural rubbers, synthetic rubbers such as polyisobutylenes, polyisoprenes, styrene block copolymers and silicone polymers, with polyisobutylenes being particularly preferred.

A transdermal drug delivery composition of the invention can be used to induce an analgesic effect. To provide the desired analgesia, the composition is placed on the skin and allowed to remain for a time sufficient to achieve or maintain the intended analgesic effect. The time that constitutes a sufficient time can be selected by those skilled in the art with consideration of the flux rate provided by of the device of the invention and of the condition being treated.

The following examples are provided to further illustrate the invention, but are not intended to limit the invention in any way. Concentrations of fentanyl and adjuvants are given as the percent by weight based on the total weight of the composition. Concentrations of A monomer, B monomer, and macromonomer are given as the percent by weight based on the charge ratios used in the polymer synthesis.
The present invention is summarized by the following items:
1. A transdermal drug delivery composition comprising
   (a) a copolymer comprising
      (i) one or more A monomers selected from the group consisting of alkyl acrylates containing 4 to 12 carbon atoms in the alkyl group and alkyl methacrylates containing 4 to 12 carbon atoms in the alkyl group; and
      (ii) one or more ethylenically unsaturated B monomers copolymerizable with the A monomer; and
   (b) about 8% to about 30% by weight fentanyl based on the total weight of the composition.
2. The composition of item 1 wherein the A monomer is selected from the group consisting of isooctyl acrylate, 2-ethylhexyl acrylate, butyl acrylate, and cyclohexyl acrylate.
3. The composition of item 1 wherein the A monomer is isooctyl acrylate.
4. The composition of item 1 wherein the B monomer is selected from the group consisting of 2-hydroxyethyl acrylate, 2-hydroxyethyl methacrylate, glyceryl acrylate, N, N-diethylacrylamide, 2-ethoxyethoxyethyl acrylate, 2-ethoxyethyl acrylate, tetrahydrofurfuryl acrylate, acrylic acid, acrylamide, vinyl acetate, N-vinyl pyrrolidone and mixtures thereof.
5. The composition of item 1 wherein the B monomer is 2-hydroxyethyl acrylate.
6. The composition of item 5 wherein the copolymer comprises from about 5% to about 45% of 2-hydroxyethyl acrylate by weight based on the total weight of all monomers in the copolymer.
7. The composition of item 1 wherein the copolymer further comprises a macromonomer.
8. The composition of item 7 wherein the macromonomer is a functionally terminated polymethylmethacrylate.
9. The composition of item 7 wherein the copolymer contains from about 1% to about 6% of macromonomer by weight based on the total weight of all monomers in the copolymer.
10. The composition of item 1 wherein the composition further comprises a delivery enhancing adjuvant.
11. The composition of item 10 wherein the delivery enhancing adjuvant is selected from the group consisting of alkane polyols, fatty acids, fatty acid esters, fatty alcohols, terpenes, C₅-C₁₈ alkyl esters of a carboxylic acid, and mixtures thereof.
12. The composition of item 10 wherein the delivery enhancing adjuvant is selected from the group consisting of ethyl oleate, isopropyl myristate, glycerol, tetraglycol, methyl laurate, N,N-dimethyldodecylamine N-oxide, limonene, terpineol, tetraethylene glycol, menthol, and mixtures thereof.
13. The composition of item 10 wherein the concentration of skin permeation enhancer is from about 5% to about 40% by weight based on the total weight of the composition.
14. The composition of item 10 wherein the skin permeation enhancer is tetraglycol.
15. The composition of item 10 wherein the skin permeation enhancer is methyl laurate.
16. The composition of item 1 wherein the concentration of fentanyl in said transdermal drug delivery composition is from about 12% to about 24% by weight.
17. The composition of item 7 wherein the copolymer comprises from about 50 to about 94% isooctyl acrylate, about 5% to about 40% 2-hydroxyethyl acrylate, about 1% to about 6% macromonomer, and 0% to about 20% vinyl acetate by weight.
18. The composition of item 7 wherein the copolymer comprises from about 52% to about 60% isooctyl acrylate, about 35% to about 40% 2-hydroxyethyl acrylate, about 1% to about 4% macromonomer, and 0% to about 10% vinyl acetate by weight.
19. The composition of item 17 wherein the concentration of fentanyl is from about 12% to about 22% by weight, wherein the composition further comprises about 15% to about 35% by weight of a permeation enhancer selected from the group consisting of methyl laurate, tetraglycol, and mixtures thereof.
20. The composition of item 19 wherein the concentration of fentanyl is from about 12% to about 17% by weight and the concentration of methyl laurate is from about 20% to about 35% by weight.
21. The composition of item 19 wherein the concentration of fentanyl is from about 15% to about 22% by weight and the concentration of tetraglycol is from about 15% to about 25% by weight.
22. The composition of item 1 wherein the composition is substantially free of undissolved fentanyl.
23. A pressure sensitive adhesive composition for the transdermal delivery of fentanyl comprising:
   (a) a polymer;
   (b) fentanyl; and
   (c) a delivery enhancing adjuvant selected from the group consisting of methyl laurate, tetraglycol, and mixtures thereof.
24. The composition of item 23 wherein the concentration of delivery enhancing adjuvant is from about 5% to about 40% by weight based on the total weight of the composition.
25. The composition of item 23 wherein the pressure sensitive adhesive copolymer comprises a copolymer comprising
   (a) one or more A monomers selected from the group consisting of alkyl acrylates containing 4 to 12 carbon atoms in the alkyl group and alkyl methacrylates containing 4 to 12 carbon atoms in the alkyl group; and
   (b) one or more ethylenically unsaturated B monomers copolymerizable with the A monomer.
26. The composition of item 25 wherein the B monomer is selected from the group consisting of 2-hydroxyethyl acrylate, 2-hydroxyethyl methacrylate, glyceryl acrylate, N,N-diethylacrylamide, 2-ethoxyethoxyethyl acrylate, 2-ethoxyethyl acrylate, tetrahydrofurfuryl acrylate, N-vinyl pyrrolidone and mixtures thereof.
27. A device for the transdermal delivery of fentanyl comprising a backing and a composition according to item 1, said composition being adhered to one surface of the backing.
28. A method of treating in a mammal a condition capable of treatment by fentanyl comprising the steps of:
   (a) providing a composition according to item 1;
   (b) placing the composition on the skin of a mammal; and
   (c) allowing the composition to remain on the skin for a time sufficient to establish or maintain a therapeutically effective blood level of fentanyl in the mammal.
29. A method of providing analgesia to a mammal comprising the steps of:
   (a) providing a composition according to item 1;
   (b) placing the composition on the skin of a mammal; and
   (c) allowing the composition to remain on the skin for a time sufficient to establish or maintain an analgesically effective blood level of fentanyl in the mammal.
30. A method of providing sustained analgesia to a mammal comprising delivering fentanyl to a mammal via a transdermal drug delivery device in an amount of about 0.5 to about 5.0 mg/day thereby causing the serum concentration of fentanyl in the mammal to be about 0.2 to about 10 ng/mL for a period of time from about 4 to about 14 days.
31. The method of item 30 wherein the fentanyl is delivered in an amount of 0.5 to 2.5 mg/day, the serum concentration of fentanyl in the mammal is about 0.3 to about 4 ng/mL, and the period of time is from about 6 to about 8 days.
32. A device for the transdermal delivery of fentanyl comprising:
   (a) a drug reservoir layer comprising the composition of item 1;
   (b) a rate controlling membrane adhered to one surface of the drug reservoir layer; and
   (c) a skin contacting pressure sensitive adhesive layer adhered to the surface of the membrane that is opposed to the surface of the membrane in contact with the reservoir layer.
33. A device for the transdermal delivery of fentanyl comprising:
   (a) a drug reservoir layer comprising the composition of item 17;
   (b) a rate controlling membrane adhered to one surface of the drug reservoir layer; and
   (c) a skin contacting pressure sensitive adhesive layer adhered to the surface of the membrane that is opposed to the surface of the membrane in contact with the reservoir layer.

### Examples

### In Vitro Skin Permeation Test Method

The skin permeation data given in the examples below was obtained using the following test method. When a transdermal delivery device was evaluated, the release liner was removed from a 2.0 cm² patch and the patch was applied to human cadaver skin and pressed to cause uniform contact with the skin. The resulting patch/skin laminate was placed patch side up across the orifice of the lower portion of a vertical diffusion cell. The diffusion cell was assembled and the lower portion filled with 10 mL of warm (32°C) receptor fluid (0.1 M phosphate buffer, pH 6.8) so that the receptor fluid contacted the skin. The sampling port was covered except when in use.

The cells were maintained at 32 ± 2°C throughout the course of the experiment. The receptor fluid was stirred by means of a magnetic stirrer throughout the experiment to assure a uniform sample and a reduced diffusion barrier on the dermal side of the skin. The entire volume of receptor fluid was withdrawn at specified time intervals and immediately replaced with fresh fluid. The withdrawn fluid was filtered through a 0.45 µm filter. The last 1-2 mL were then analyzed for fentanyl using conventional high performance liquid chromatography methods (Column: Phenomenex Spherex, 75 x 4.6 mm, 3 µm particle size; Mobile phase: 400:200:400 Methanol:Acetonitrile:Buffer. Buffer is ammonium acetate solution adjusted to pH 6.6 with acetic acid; Flow Rate: 2 mL/min; Detector: uv at 230 nm; Injection Volume: 10 µL; Run time: 1.9 minutes). The cumulative amount of fentanyl penetrating through the skin was calculated and reported as µg/cm².

### Stability Test Method

Transdermal drug delivery devices (20 cm² patches) were sealed in pouches (BAREX™/aluminum/polyester or BAREX™/aluminum/paper laminates) and stored under conditions of 25°C/60 % relative humidity and 40°C/75 % relative humidity. The patches were tested for their drug content and/or their probe tack before storage and after preset storage times using the test methods described below.

### Drug Content Test Method

The drug content data given in the examples below was obtained using the following test method. The liner was removed from the patches and the patches were placed in a 120 mL jar. The backing and coating were extracted using 75 mL of a solution consisting of 75:25 by volume tetrahydrofuran (THF):methanol (MeOH). The samples were allowed to shake overnight. Dilutions of the samples were then prepared by placing 10 mL of the resulting solutions into 44 mL vials and adding 30 mL additional THF:MeOH to each vial. Aliquots of these final dilutions were then placed in autosampler vials for analysis. Analysis of the samples was performed by gas chromatography with flame ionization detection (GC-FID) using a J&W DB-5 fused silica capillary column (15 m x 0.53 mm i.d., 1.5 µm film of (5%-Phenyl)-methylpolysiloxane) with helium carrier gas.

### Probe Tack Test Method

The tack data given in the examples below was obtained using a Digital Polyken Probe Tack Tester, Model 80-02-01 (Testing Machines, Inc., Amityville, NY). The machine settings were as follows: speed: 0.5 cm/second, dwell: 2 seconds; mode: peak. A stainless steel probe was used. The result of the test is the force required to break the bond between the probe and the surface of the test sample. The force is measured in "grams of tack".

### Peel Adhesion to Vitro-Skin Method

The peel adhesion data given in the examples below was obtained using the following test method. The peel adhesion testing was based on ASTM D3330-90. This involves peel from a substrate at a 180° peel angle done with a constant-rate-of-extension tensile tester. The substrate used was Vitro-Skin™N-19 (VS), an artificial skin substitute available from Innovative Measurement Solutions, Inc., that is designed to mimic human back skin.

The following modifications to the ASTM test method were necessary in order to use VS as a test substrate. The VS was conditioned prior to use at 23°C for at least 16 hours in a chamber containing a solution consisting of 70:30 by volume water:glycerol to maintain a constant humidity. All testing was done with the textured side of the VS. Immediately upon removal of the VS from the conditioning chamber, the VS was attached using a double-sided adhesive tape to the backing of a foam tape (3M 1777, 40 mil (1016 µm) thick) which was attached to a steel plate to provide a stable testing surface. Testing was done in a controlled environment at 23°C ± 2°C and 50% ± 3% relative humidity. A 1.0 cm width strip of a coated sheet was then applied to the VS and rolled down with one pass of a standard 2.04 kg rubber roller. After rolldown, the 1.0 cm width strip of the coated sheet was allowed to dwell for 2 minutes prior to peel testing.

The free end of the coated strip was doubled back so that the angle of removal was 180°. The removal peel rate was 6 inches/minute (15.2 cm/minute). The force of adhesion is reported as grams per centimeter (g/cm). It is noted in the results when the adhesive showed cohesive failure (i.e., splitting of the adhesive upon removal).

### Preparation of the Copolymers

The copolymers used in the examples that follow were prepared generally according to the methods described below. The inherent viscosity values which are reported below were measured by conventional means using a Cannon-Fenske #50 viscometer in a water bath controlled at 27°C to measure the flow time of 10 millimeters of the polymer solution (0.15 g of polymer per deciliter of ethyl acetate). The test procedure and apparatus are described in detail in Textbook of Polymer Science, F.W. Billmeyer, Wiley Interscience, Second Edition (1971), pages 84 and 85.

### Preparation of "Dried" Copolymer

Dried copolymer was prepared by knife coating a solution of the copolymer onto a release liner. The coated release liner was oven dried to remove the solvent and reduce the level of residual monomers. The dried copolymer was then stripped off of the release liner and stored in a container until used.

### Copolymer A. Preparation of Isooctyl Acrylate/2-Hydroxyethyl acrylale/Elvacite™ 1010 (58/39/3) Copolymer

A master batch was prepared by combining isooctyl acrylate (626.4 g), 2-hydroxyethyl acrylate (421.2 g), polymethylmethacrylate macromonomer (32.4 g of ELVACITE™ 1010 available from ICI Acrylics), 2,2'-azobis(2-methylbutyronitrile) (2.16 g), ethyl acetate (1555.2 g) and isopropanol (64.8 g). The resulting solution was divided in equal portions and placed into six 1 quart (0.95 L) amber glass bottles. The bottles were purged for 2 minutes with nitrogen at a flow rate of 1 L per minute. The bottles were sealed and placed in a rotating water bath at 57°C for 24 hours. At 24 hours the bottles were removed from the rotating water bath, unsealed, and recombined into a 1 gallon (3.8 L) glass jar. The percent solids of the resultant copolymer was 38.1 %. The inherent viscosity was 0.88 dL/g.

### Copolymer B. Preparation of Isooctyl Acrylate/2-Hydroxyethyl acrylate/Vinyl Acetate /Elvacite™ 1010 (62/15/20/3) Copolymer

A master batch was prepared by combining isooctyl acrylate (714.24 g), 2-hydroxyethyl acrylate (172.8 g), polymethylmethacrylate macromonomer (34.56 g of ELVACITE™ 1010 available from ICI Acrylics), vinyl acetate (230.4 g), 2,2'-azobis(2-methylbutyronitrile) (2.304 g), ethyl acetate (1210.56 g) and isopropanol (37.44 g). The resulting solution was divided in equal portions and placed into six 1 quart (0.95 L) amber glass bottles. The bottles were purged for 2 minutes with nitrogen at a flow rate of 1 L per minute. The bottles were sealed and placed in a rotating water bath at 55°C for 24 hours. At 24 hours the bottles were removed from the rotating water bath, unsealed, and recombined into a 1 gallon (3.8 L) glass jar. The percent solids of the resultant copolymer was 40.4%. The inherent viscosity was 1.13 dL/g.

### Copolymer C. Preparation of Isooctyl Acrylate/2-Hydroxyethyl acrylate/Elvacite™ 1010/Vinyl Acetate (60/39/1/10) Copolymer

A solution was prepared by combining isooctyl acrylate (150.0 g), 2-hydroxyethyl acrylate (97.5 g), polymethylmethacrylate macromonomer (2.5 g of ELVACITE™ 1010 available from ICI Acrylics), 2,2'-azobis(2-methylbutyronitrile) (0.375 g), ethyl acetate (327.98 g) and isopropanol (17.26 g) in a 1 quart (0.95 L) amber glass bottle. The bottle was purged for 2 minutes with nitrogen at a flow rate of 1 L per minute. The bottle was sealed and placed in a rotating water bath at 57°C for 24 hours. At 24 hours the bottle was removed from the rotating water bath and unsealed. Vinyl acetate (25.0 g) and an additional charge of 2,2'-azobis(2-methylbutyronitrile) (0.25 g) were added to the bottle. The bottle was purged for 2 minutes with nitrogen at a flow rate of 1 L per minute. The bottle was sealed and placed in a rotating water bath at 57°C for an additional 24 hours. At 24 hours the bottle was removed from the rotating water bath and unsealed. The resulting copolymer was diluted with ethyl acetate (115.90 g) and isopropanol (9.40 g) to 32.7% solids. The inherent viscosity was 0.98 dL/g.

### Copolymer D. Preparation of Isooctyl Acrylate/2-Hydroxyethyl acrylate/Elvacite™ 1010/Vinyl Acetate (58.5/39/2.5/10) Copolymer

A solution was prepared by combining isooctyl acrylate (146.25 g), 2-hydroxyethyl acrylate (97.5 g), polymethylmethacrylate macromonomer (6.25 g of ELVACITE™ 1010 available from ICI Acrylics), 2,2'-azobis(2-methylbutyronitrile) (0.375 g), ethyl acetate (356.25 g) and isopropanol (18.75 g) in a 1 quart (0.95 L) amber glass bottle. The bottle was purged for 2 minutes with nitrogen at a flow rate of 1 L per minute. The bottle was sealed and placed in a rotating water bath at 57°C for 24 hours. At 24 hours the bottle was removed from the rotating water bath and unsealed. Vinyl acetate (25.0 g) and an additional charge of 2,2'-azobis(2-methylbutyronitrile) (0.25 g) were added to the bottle. The bottle was purged for 2 minutes with nitrogen at a flow rate of 1 L per minute. The bottle was sealed and placed in a rotating water bath at 57°C for an additional 24 hours. At 24 hours the bottle was removed from the rotating water bath and unsealed. The percent solids of the resultant copolymer was 39.6%. The inherent viscosity was 0.85 dL/g.

### Copolymer E. Preparation of Isooctyl Acrylate/2-Hydroxyethyl acrylate/Elvacite™ 1010/Vinyl Acetate (57/39/4/10) Copolymer

A solution was prepared by combining isooctyl acrylate (142.5 g), 2-hydroxyethyl acrylate (97.5 g), polymethylmethacrylate macromonomer (10.0 g of ELVACITE™ 1010 available from ICI Acrylics), 2,2'-azobis(2-methylbutyronitrile) (0.375 g), ethyl acetate (327.98 g) and isopropanol (17.25 g) in a 1 quart (0.95 L) amber glass bottle. The bottle was purged for 2 minutes with nitrogen at a flow rate of 1 L per minute. The bottle was sealed and placed in a rotating water bath at 57°C for 24 hours. At 24 hours the bottle was removed from the rotating water bath and unsealed. Vinyl acetate (25.0 g) and an additional charge of 2,2'-azobis(2-methylbutyronitrile) (0.25 g) were added to the bottle. The bottle was purged for 2 minutes with nitrogen at a flow rate of 1 L per minute. The bottle was sealed and placed in a rotating water bath at 57°C for an additional 24 hours. At 24 hours the bottle was removed from the rotating water bath and unsealed. The resulting copolymer was diluted with ethyl acetate (113.10 g) and isopropanol (5.95 g) to 34.4% solids. The inherent viscosity was 0.61 dL/g.

### Copolymer F. Preparation of Isooctyl Acrylate/2-Hydroxyethyl acrylate/Elvacite™ 1010/Vinyl Acetate (57/39/4/10) Copolymer

A master batch was prepared by combining isooctyl acrylate (641.25 g), 2-hydroxyethyl acrylate (438.75 g), polymethylmethacrylate macromonomer (45.0 g of ELVACITE™ 1010 available from ICI Acrylics), 2,2'-azobis(2-methylbutyronitrile) (1.6875 g), ethyl acetate (1360.215 g) and isopropanol (71.590 g). A portion (568.55 g) of the resulting solution was placed in a 1 quart (0.95 L) amber glass bottle. The bottle was purged for 2 minutes with nitrogen at a flow rate of 1 L per minute. The bottle was sealed and placed in a rotating water bath at 55°C for 16 hours. The temperature of the rotating water bath was then increased to 57°C for an additional 8 hours. At 24 hours the bottle was removed from the rotating water bath and unsealed. Vinyl acetate (25.0 g) and an additional charge of 2,2'-azobis(2-methylbutyronitrile) (0.25 g) were added to the bottle. The bottle was purged for 2 minutes with nitrogen at a flow rate of I L per minute. The bottle was sealed and placed in a rotating water bath at 57°C for an additional 24 hours. The percent solids of the resultant copolymer was 43.9%. The inherent viscosity was 0.76 dL/g.

### Copolymer G. Preparation of Isooctyl Acrylate/Vinyl Acetate/Elvacite™ 1010 (56/38/6) Copolymer

A master batch was prepared by combining isooctyl acrylate (574.56 g), vinyl acetate (389.88 g), polymethylmethacrylate macromonomer (61.56 g of ELVACITE™ 1010 available from ICI Acrylics), 2,2'-azobis(2-methylbutyronitrile.) (2.0525 g), and ethyl acetate (1674.0 g). The resulting solution was divided in equal portions and placed into six 1 quart (0.95 L) amber glass bottles. The bottles were purged for 2 minutes with nitrogen at a flow rate of 1 L per minute. The bottles were sealed and placed in a rotating water bath at 57°C for 24 hours. At 24 hours the bottles were removed from the rotating water bath, unsealed, and recombined into a 1 gallon (3.8 L) glass jar. The percent solids of the resultant copolymer was 27.6%. The inherent viscosity was 0.80 dL/g.

### Copolymer H. Preparation of Isooctyl Acrylate/Acrylamide/Vinyl Acetate (75/5/20) Copolymer

A master batch was prepared by combining isooctyl acrylate (621.0 g), acrylamide (41.4 g), vinyl acetate (165.6 g), 2,2'-azobis(2,4-dimethylpentanenitrile) (1.656 g), ethyl acetate (884.5 g) and methanol (87.48 g). A portion (400 g) of the resulting solution was placed in a 1 quart (0.95 L) amber glass bottle. The bottle was purged for 2 minutes with nitrogen at a flow rate of 1 L per minute. The bottle was sealed and placed in a rotating water bath at 45°C for 24 hours. The resulting copolymer was diluted with ethyl acetate (183.6 g) and methanol (20.4 g) to 30.5% solids. The inherent viscosity was 1.39 dL/g.

### Example 1

Fentanyl (1.4014 g) was added to methanol (6.0056 g) and mixed until all of the fentanyl was dissolved. To this solution, copolymer (8.6788 g of dried isooctyl acrylate/2-hydroxyethyl acrylate/Elvacite™ 1010 (58/39/3) from Copolymer A above) and ethyl acetate (24.0541 g) were added and mixed until a uniform coating formulation was obtained. The coating formulation was knife coated at a wet thickness of 24 mil (609.6 µm) onto a release liner (Daubert 164P silicone coated release liner). The coated liner was oven dried for 4 minutes at 110°F (43°C), for 2 minutes at 185°F (85°C), and for 2 minutes at 225°F (107°C). The resulting coating contained 13.9 percent fentanyl. The coated liner was laminated onto a backing (SCOTCHPAK™ 1012 polyester film laminate; available from 3M Company). The permeation through human cadaver skin was determined using the test method described above. The results are shown in Table I below. Results of stability testing of fentanyl content and probe tack force were determined using the test methods described above. The results are shown in Table 2 below.

### Example 2

Fentanyl (0.5589 g) was added to methanol (3.0770 g) and mixed until all of the fentanyl was dissolved. To this solution, copolymer (2.9409 g of dried isooctyl acrylate/2-hydroxyethyl acrylate/Elvacite™ 1010 (58/39/3) from Copolymer A above), methyl laurate (1.5602 g), and ethyl acetate (12.0039 g) were added and mixed until a uniform coating formulation was obtained. The coating formulation was knife coated at a wet thickness of 24 mil (609.6 µm) onto a release liner (Daubert 164P silicone coated release liner). The coated liner was oven dried for 4 minutes at 110°F (43°C), for 2 minutes at 185°F (85°C), and for 2 minutes at 225°F (107°C). The resulting coating contained 11.0 percent fentanyl and 30.8 percent methyl laurate. The coated liner was laminated onto a backing (SCOTCHPAK™ 1012 polyester film laminate; available from 3M Company). The permeation through human cadaver skin was determined using the test method described above. The results are shown in Table 1 below.

### Example 3

Fentanyl (0.4964 g) was added to methanol (3.00468 g) and mixed until all of the fentanyl was dissolved. To this solution, copolymer (3.0096 g of dried isooctyl acrylate/2-hydroxyethyl acrylate/Elvacite™ 1010 (58/39/3) from Copolymer A above), isopropyl myristate (1.5094 g), and ethyl acetate (12.0550 g) were added and mixed until a uniform coating formulation was obtained. The coating formulation was knife coated at a wet thickness of 24 mil (609.6 µm) onto a release liner (Daubert 164P silicone coated release liner). The resulting coated liner was oven dried for 4 minutes at 110°F (43°C), for 2 minutes at 185°F (85°C), and for 2 minutes at 225°F (107°C). The resulting coating contained 9.9 percent fentanyl and 30.1 percent isopropyl myristate. The coated liner was laminated onto a backing (SCOTCHPAK™ 1012 polyester film laminate; available from 3M Company). The permeation through human cadaver skin was determined using the test method described above. The results are shown in Table 1 below.

### Example 4

Fentanyl (1.4010 g) was added to methanol (6.0567 g) and mixed until all of the fentanyl, was dissolved. To this solution, copolymer (8.5966 g of dried isooctyl acrylate/2-hydroxyethyl acrylate/vinyl acetate /Elvacite™ 1010 (62/15/20/3) from Copolymer B above) and ethyl acetate (24.0166 g) were added and mixed until a uniform coating formulation was obtained. The coating formulation was knife coated at a wet thickness of 24 mil (609.6 µm) onto a release liner (Daubert 164P silicone coated release liner). The resulting coated liner was oven dried for 4 minutes at 110°F (43°C), for 2 minutes at 185°F (85°C), and for 2 minutes at 225°F (107°C) and then it was laminated onto a backing (SCOTCHPAK™ 1012 polyester film laminate; available from 3M Company). The resulting coating contained 14.0 percent fentanyl. The permeation through human cadaver skin was determined using the test method described above. The results are shown in Table 1 below. Results of stability testing of fentanyl content and probe tack force were determined using the test methods described above. The results are shown in Table 2 below.

### Example 5

Fentanyl (0.5580 g) was added to methanol (3.0036 g) and mixed until all of the fentanyl was dissolved. To this solution, copolymer (2.9409 g of dried isooctyl acrylate/2-hydroxyethyl acrylate/vinyl acetate /Elvacite™ 1010 (62/15/20/3) from Copolymer B above), methyl laurate (1.5020 g), and ethyl acetate (12.8355 g) were added and mixed until a uniform coating formulation was obtained. The coating formulation was knife coated at a wet thickness of 24 mil (609.6 µm) onto a release liner (Daubert 164P silicone coated release liner). The resulting coated liner was oven dried for 4 minutes at 110°F (43°C), for 2 minutes at 185°F (85°C), and for 2 minutes at 225°F (107°C) and then it was laminated onto a backing (SCOTCHPAK™ 1012 polyester film laminate; available from 3M Company). The resulting coating contained 11.2 percent fentanyl and 30.0 percent methyl laurate. The permeation through human cadaver skin was determined using the test method described above. The results are shown in Table I below.

### Example 6

Fentanyl (0.4950 g) was added to methanol (3.0217 g) and mixed until all of the fentanyl was dissolved. To this solution, copolymer (3.0268 g of dried isooctyl acrylate/2-hydroxyethyl acrylate/vinyl acetate /Elvacite™ 1010 (62/15/20/3) from Copolymer B above), isopropyl myristate (1.5009 g), and ethyl acetate (12.1759 g) were added and mixed until a uniform coating formulation was obtained. The coating formulation was knife coated at a wet thickness of 24 mil (609.6 µm) onto a release liner (Daubert 164P silicone coated release liner). The resulting coated liner was oven dried for 4 minutes at 110°F (43°C), for 2 minutes at 185°F (85°C), and for 2 minutes at 225°F (107°C) and then it was laminated onto a backing (SCOTCHPAK™ 1012 polyester film laminate; available from 3M Company). The resulting coating contained 9.9 percent fentanyl and 29.9 percent isopropyl myristate. The permeation through human cadaver skin was determined using the test method described above. The results are shown in Table 1 below.

| Table 1 | | | | | | | | | | | |
|---|---|---|---|---|---|---|---|---|---|---|---|
| Human Cadaver Skin Permeation | | | | | | | | | | | |
| Example Number | Average Cumulative Amount Penetrated (µg/cm²) | | | | | | | | | | |
| | 2 hr | 4 hr | 8 hr | 12 hr | 24 hr | 48 hr | 72 hr | 96 hr | 120 hr | 144 hr | 168 hr |
| 1 | 2 | 5 | 19 | 34 | 91 | 218 | 333 | 431 | 516 | 586 | 654 |
| 2 | 2 | 5 | 24 | 46 | 126 | 282 | 399 | 468 | 512 | 541 | 562 |
| 3 | 1 | 2 | 10 | 20 | 66 | 178 | 293 | 385 | 464 | 527 | 583 |
| 4 | 2 | 5 | 18 | 33 | 94 | 234 | 377 | 498 | 611 | 708 | 801 |
| 5 | 7 | 18 | 56 | 93 | 218 | 429 | 572 | 661 | 721 | 763 | 799 |
| 6 | 3 | 7 | 31 | 57 | 153 | 337 | 503 | 623 | 722 | 798 | 860 |

| Table 2 | | | | | | | | | | | | | |
|---|---|---|---|---|---|---|---|---|---|---|---|---|---|
| Stability Testing - Probe Tack, Fentanyl Content | | | | | | | | | | | | | |
| | | Probe Tack [grams] | | | | | | Fentanyl Content [%] | | | | | |
| Example Number | Storage Condition | Initial | 2 wk | 5 wk | 2 mo | 3 mo | 6 mo | Initial | 2 wk | 5 wk | 2 mo | 3 mo | 6 mo |
| 1 | 25/60 | 1210 | 1155 | 1031 | 1285 | 1239 | 1138 | 13.9* | - | 13.4 | 13.2 | 13.3 | - |
| - | 40/75 | - | 1258 | 1137 | 994 | 1161 | 1239 | - | - | 13.4 | 13.6 | 13.3 | - |
| 4 | 25/60 | 1186 | 1112 | 705 | 1071 | 1158 | 1193 | 14.0* | - | 13.9 | 13.8 | 13.7 | - |
| - | 40/75 | - | 1039 | 1097 | 935 | 1228 | 1249 | - | - | 13.1 | 14.0 | 13.6 | - |

| | | | | | | | | | | | | | |
|---|---|---|---|---|---|---|---|---|---|---|---|---|---|
| *Initial values are nominal fentanyl percentages based on the amounts added to the formulation. | | | | | | | | | | | | | |

### Example 7

Fentanyl (0.3508 g) was added to methanol (1.5426 g) and mixed until all of the fentanyl was dissolved. To this solution, copolymer (2.1536 g of dried isooctyl acrylate/2-hydroxyethyl acrylate/Elvacite™ 1010 (58/39/3) from Copolymer A above) and ethyl acetate (6.0006 g) were added and mixed until a uniform coating formulation was obtained. The coating formulation was knife coated at a wet thickness of 19 mil (482.6 µm) onto a release liner (Daubert 164P silicone coated release liner). The resulting coated liner was oven dried for 4 minutes at 110°F (43°C), for 2 minutes at 185°F (85°C), and for 2 minutes at 225°F (107°C) and then it was laminated onto a backing (SCOTCHPAK™ 9732 polyester film laminate; available from 3M Company). The permeation through human cadaver skin was determined using the test method described above. The results are shown in Table 4 below.

### Example 8

Fentanyl (0.3382 g) was added to methanol (1.5075 g) and mixed until all of the fentanyl was dissolved. To this solution, copolymer (1.7869 g of dried isooctyl acrylate/2-hydroxyethyl acrylate/Elvacite™ 1010 (58/39/3) from Copolymer A above), limonene (0.3737 g), and ethyl acetate (5.9952 g) were added and mixed until a uniform coating formulation was obtained. The coating formulation was knife coated at a wet thickness of 19 mil (482.6 µm) onto a release liner (Daubert 164P silicone coated release liner). The resulting coated liner was oven dried for 4 minutes at 110°F (43°C), for 2 minutes at 185°F (85°C), and for 2 minutes at 225°F (107°C) and then it was laminated onto a backing (SCOTCHPAK™ 9732 polyester film laminate; available from 3M Company). The permeation through human cadaver skin was determined using the test method described above. The results are shown in Table 4 below.

### Examples 9-15

Using the general method of Example 8, a series of transdermal delivery devices in which the amount of fentanyl and the amount and choice of adjuvant were varied was prepared. In all instances the copolymer used was isooctyl acrylate/2-hydroxyethyl acrylate/Elvacite™ 1010 (58/39/3) from Copolymer A above. The weight percent of fentanyl, weight percent of adjuvant, and identity of adjuvant are given in Table 3 below. The balance of each formulation to 100 weight percent was copolymer. The abbreviations LI, MLA, PG, and ML are used for limonene, methyl lactate, propylene glycol, and methyl laurate respectively. The permeation through human cadaver skin was determined using the test method described above. The results are shown in Table 4 below.

| Table 3 | | |
|---|---|---|
| Example Number | % Fentanyl | Adjuvant |
| 7 | 14.0 | none |
| 8 | 13.5 | 15.0% LI |
| 9 | 13.1 | 30.1%LI |
| 10 | 18.4 | 15.6% MLA |
| 11 | 23.0 | 30.3% MLA |
| 12 | 13.3 | 14.9% ML |
| 13 | 12.6 | 30.1% ML |
| 14 | 12.5 | 16.4%PG |
| 15 | 11.6 | 30.0%PG |

| Table 4 | | | | | | | | | | | |
|---|---|---|---|---|---|---|---|---|---|---|---|
| Human Cadaver Skin Permeation | | | | | | | | | | | |
| Example Number | Average Cumulative Amount Penetrated (µg/cm²) | | | | | | | | | | |
| | 3 hr | 6 hr | 12 hr | 24 hr | 30 hr | 48 hr | 54 hr | 72 hr | 78 hr | 96 hr | 120 hr |
| 7 | 13 | 26 | 51 | 110 | 138 | 233 | 266 | 346 | 429 | 527 | 693 |
| 8 | 19 | 39 | 75 | 157 | 208 | 322 | 362 | 447 | 559 | 652 | 795 |
| 9 | 17 | 25 | 42 | 87 | 115 | 196 | 222 | 287 | 380 | 459 | 594 |
| 10 | 13 | 19 | 34 | 75 | 106 | 197 | 228 | 301 | 406 | 496 | 661 |
| 11 | 10 | 11 | 12 | 27 | 38 | 85 | 103 | 146 | 211 | 273 | 386 |
| 12 | 14 | 15 | 19 | 39 | 55 | 114 | 132 | 179 | 252 | 318 | 430 |
| 13 | 11 | 14 | 20 | 45 | 62 | 129 | 153 | 207 | 285 | 357 | 496 |
| 14 | 44 | 52 | 62 | 89 | 106 | 164 | 183 | 231 | 305 | 369 | 478 |
| 15 | 18 | 32 | 40 | 66 | 83 | 140 | 168 | 216 | 290 | 350 | 449 |

### Example 16

Fentanyl (0.2987 g) was added to methanol (1.5008 g) and mixed until all of the fentanyl was dissolved. To this solution, copolymer (1.8276 g of dried isooctyl acrylate/2-hydroxyethyl acrylate/Elvacite™ 1010 (58/39/3) from Copolymer A above), polyethylene glycol (0.4849 g), and ethyl acetate (6.0052 g) were added and mixed until a uniform coating formulation was obtained. The coating formulation was knife coated at a wet thickness of 20 mil (508.0 µm) onto a release liner (Daubert 164P silicone coated release liner). The resulting coated liner was oven dried for 4 minutes at 110°F (43°C), for 2 minutes at 185°F (85°C), and for 2 minutes at 225°F (107°C) and then it was laminated onto a backing (SCOTCHPAK™ 9732 polyester film laminate; available from 3M Company). The permeation through human cadaver skin was determined using the test method described above. The results are shown in Table 6 below.

### Examples 17-21

Using the general method of Example 16, a series of transdermal delivery devices in which the amount of fentanyl and the amount and choice of adjuvant were varied was prepared. In all instances the copolymer used was isooctyl acrylate/2-hydroxyethyl acrylate/Elvacite™ 1010 (58/39/3) from Copolymer A above. The weight percent of fentanyl, weight percent of adjuvant, and identity of adjuvant are given in Table 5 below. The balance of each formulation to 100 weight percent was copolymer. The abbreviations PEG, TG, and TEG are used for polyethylene glycol 400 (Carbowax® PEG 400), tetraglycol, and tetraethylene glycol respectively. The permeation through human cadaver skin was determined using the test method described above. The results are shown in Table 6 below.

| Table 5 | | |
|---|---|---|
| Example Number | % Fentanyl | Adjuvant |
| 16 | 11.4 | 18.6% PEG |
| 17 | 9.8 | 30.1% PEG |
| 18 | 11.8 | 15.9% TG |
| 19 | 9.8 | 30.1% TG |
| 20 | 11.6 | 16.9% TEG |
| 21 | 9.5 | 31.8% TEG |
| 22 | 11.8 | 16.0% MTH |
| 23 | 9.7 | 30.7% MTH |
| 24 | 11.6 | 17.0% TP |
| 25 | 9.8 | 30.1% TP |

| Table 6 | | | | | | | | | | | | |
|---|---|---|---|---|---|---|---|---|---|---|---|---|
| Human Cadaver Skin Permeation | | | | | | | | | | | | |
| Example Number | Average Cumulative Amount Penetrated (µg/cm²) | | | | | | | | | | | |
| | 3 hr | 6 hr | 12 hr | 24 hr | 30 hr | 48 hr | 54 hr | 72 hr | 96 hr | 120 hr | 144 hr | 168 hr |
| 16 | 1 | 1 | 7 | 34 | 46 | 86 | 105 | 141 | 193 | 236 | 274 | 310 |
| 17 | 0 | 0 | 5 | 26 | 37 | 75 | 91 | 121 | 161 | 199 | 232 | 262 |
| 18 | 0 | 4 | 27 | 90 | 113 | 185 | 212 | 263 | 324 | 368 | 402 | 430 |
| 19 | 0 | 8 | 41 | 126 | 159 | 266 | 305 | 389 | 490 | 563 | 613 | 652 |
| 20 | 0 | 3 | 23 | 76 | 97 | 167 | 194 | 252 | 327 | 388 | 437 | 480 |
| 21 | 1 | 3 | 17 | 68 | 90 | 163 | 192 | 253 | 334 | 398 | 449 | 493 |
| 22 | 0 | 1 | 7 | 25 | 30 | 47 | 55 | 69 | 92 | 113 | 131 | 150 |
| 23 | 0 | 1 | 7 | 23 | 31 | 46 | 52 | 64 | 84 | 104 | 121 | 138 |
| 24 | 0 | 1 | 6 | 22 | 28 | 48 | 55 | 72 | 99 | 125 | 148 | 170 |
| 25 | 0 | 2 | 10 | 31 | 39 | 64 | 74 | 96 | 132 | 166 | 196 | 225 |

### Example 22

Fentanyl (0.2985 g) was added to methanol (1.4947 g) and mixed until all of the fentanyl was dissolved. To this solution, dried copolymer (1.8214 g of isooctyl acrylate/2-hydroxyethyl acrylate/Elvacite™ 1010 (58/39/3) from Copolymer A above), menthol (0.4046 g), and ethyl acetate (6.0041 g) were added and mixed until a uniform coating formulation was obtained. The coating formulation was knife coated at a wet thickness of 24 mil (609.6 µm) onto a release liner (Daubert 164P silicone coated release liner). The resulting coated liner was oven dried for 10 minutes at 110°F (43°C) and then it was laminated onto a backing (SCOTCHPAK™ 9732 polyester film laminate; available from 3M Company). The permeation through human cadaver skin was determined using the test method described above. The results are shown in Table 6 above.

### Examples 23-25

Using the general method of Example 22, a series of transdermal delivery devices in which the amount of fentanyl and the amount and choice of adjuvant were varied was prepared. In all instances the copolymer used was isooctyl acrylate/2-hydroxyethyl acrylate/Elvacite™ 1010 (58/39/3) from Copolymer A above. The weight percent of fentanyl, weight percent of adjuvant, and identity of adjuvant are given in Table 5 above. The balance of each formulation to 100 weight percent was copolymer. The abbreviations MTH and TP are used for menthol and terpineol respectively. The permeation through human cadaver skin was determined using the test method described above. The results are shown in Table 6 above.

### Examples 26-30

Using the general method of Example 16, a series of transdermal delivery devices in which the amount of fentanyl and the amount and choice of adjuvant were varied was prepared. In all instances the copolymer used was isooctyl acrylate/2-hydroxyethyl acrylate/Elvacite™ 1010 (58/39/3) from Copolymer A above. The weight percent of fentanyl, weight percent, and identity of adjuvant(s) are given in Table 7 below. The balance of each formulation to 100 weight percent was copolymer. The abbreviations ML, TG, and LI are used for methyl laurate, tetraglycol, and limonene respectively. The permeation through human cadaver skin was determined using the test method described above. The results are shown in Table 8 below.

| Table 7 | | |
|---|---|---|
| Example Number | % Fentanyl | Adjuvant |
| 26 | 17.1 | 30.4% TG |
| 27 | 13.0 | 30.1 % LI |
| 28 | 14.2 | 10.0% ML, 10.2%TG, 10.0% LI |
| 29 | 12.6 | 30.2% ML |
| 30 | 15.1 | 14.8% TG, 15.4% LI |

| Table 8 | | | | | | | | | | |
|---|---|---|---|---|---|---|---|---|---|---|
| Human Cadaver Skin Permeation | | | | | | | | | | |
| Example Number | Average Cumulative Amount Penetrated (µg/cm²) | | | | | | | | | |
| | 3 hr | 6 hr | 12 hr | 24 hr | 30 hr | 48 hr | 54 hr | 72 hr | 144 hr | 168 hr |
| 26 | 54 | 140 | 283 | 474 | 540 | 678 | 727 | 807 | 1007 | 1054 |
| 27 | 24 | 59 | 125 | 239 | 288 | 398 | 431 | 507 | 682 | 720 |
| 28 | 48 | 114 | 217 | 348 | 397 | 482 | 502 | 543 | 618 | 633 |
| 29 | 21 | 61 | 147 | 296 | 365 | 519 | 565 | 659 | 821 | 852 |
| 30 | 15 | 40 | 93 | 183 | 226 | 320 | 349 | 408 | 519 | 535 |

### Example 31

Fentanyl (0.6430 g) was added to methanol (0.8113 g) and mixed until all of the fentanyl was dissolved. To this solution, copolymer (2.5525 g of dried isooctyl acrylate/2-hydroxyethyl acrylate/Elvacite™ 1010/vinyl acetate (60/39/1/10) from Copolymer C above), tetraglycol (0.8002 g), and ethyl acetate (3.1933 g) were added and mixed until a uniform coating formulation was obtained. The coating formulation was knife coated at a wet thickness of 11 mil (279.4 µm) onto a release liner (Daubert 164P silicone coated release liner). The resulting coated liner was oven dried for 4 minutes at 110°F (43°C), for 2 minutes at 18S°F (85°C), and for 2 minutes at 200°F (93°C) and then it was laminated onto a backing (SCOTCHPAK™ 9732 polyester film laminate; available from 3M Company). The peel adhesion to Vitro-skin was determined using the test method described above. The results are shown in Table 9 below.

| Table 9 | | | | | |
|---|---|---|---|---|---|
| Example Number | % Fentanyl | Adjuvant | Copolymer ID | % Elvacite in copolymer | Adhesion to Vitro-Skin [g/cm] |
| 31 | 16.1 | 20.0% TG | C | 1 | 187^{a} |
| 32 | 16.8 | 25.4% TG | C | 1 | 40-120^{b} |
| 33 | 16.7 | 25.1% TG | D | 2.5 | 122^{a} |
| 34 | 16.0 | 20.2% TG | E | 4 | 260^{a} |
| 35 | 13.0 | 19.6% ML | C | 1 | 83 |
| 36 | 13.0 | 24.9% ML | C | 1 | 72 |
| 37 | 13.3 | 20.3% ML | D | 2.5 | 105 |

| | | | | | |
|---|---|---|---|---|---|
| ^{a} cohesive failure ^{b} The sample alternated between cohesive failure (high force) and adhesive failure (low force). | | | | | |

### Examples 32-37

Using the general method of Example 31, a series of transdermal delivery devices in which the amount of fentanyl, the amount of adjuvant, the choice of adjuvant, and the amount of Elvacite™1010 in the copolymer were varied was prepared. In all instances the copolymer used was isooctyl acrylate/2-hydroxyethyl acrylate/Elvacite™ 1010/vinyl acetate. The weight percent of fentanyl, weight percent adjuvant, identity of adjuvant, identity of copolymer, and weight percent of Elvacite™ 1010 in the copolymer are given in Table 9 above. The balance of each formulation to 100 weight percent was copolymer. The abbreviations ML and TG are used for methyl laurate and tetraglycol respectively. The peel adhesion to Vitro-skin was determined using the test method described above. The results are shown in Table 9 above.

### Example 38

Fentanyl (1.240 g) was added to methanol (2.993 g) and mixed until all of the fentanyl was dissolved. To this solution, copolymer (5.271 g of dried isooctyl acrylate/2-hydroxyethyl acrylate/Elvacite™ 1010/Vinyl acetate (58.5/39/2.5/10) from Copolymer D above), methyl laurate (3.506 g), and ethyl acetate (12.034 g) were added and mixed until a uniform coating formulation was obtained. The coating formulation was knife coated at a wet thickness of 20 mil (508.0 µm) onto a release liner (Daubert 164P silicone coated release liner). The resulting coated liner was oven dried for 4 minutes at 110°F (43°C), for 2 minutes at 185°F (85°C), and for 2 minutes at 200°F (93°C) and then it was laminated onto a backing (SCOTCHPAK™ 9732 polyester film laminate; available from 3M Company). The resulting coating contained 12.4 percent fentanyl and 35.0 percent methyl laurate.

### Example 39

Fentanyl (2.1994 g) was added to methanol (1.9991 g) and mixed until all of the fentanyl was dissolved. To this solution, copolymer (5.6518 g of dried isooctyl acrylate/2-hydroxyethyl acrylate/Elvacite™ 1010/vinyl acetate (57/39/4/10) from Copolymer F above), tetraglycol (2.0157 g), N, N-dimethyldodecylamine N-oxide (0.1490 g), and ethyl acetate (8.1121 g) were added and mixed until a uniform coating formulation was obtained. The coating formulation was knife coated at a wet thickness of 13 mil (330.2 µm) onto a release liner (Daubert 164P silicone coated release liner). The resulting coated liner was oven dried for 4 minutes at 110°F (43°C), for 2 minutes at 185°F (85°C), and for 2 minutes at 225°F (107°C) and then it was laminated onto a backing (SCOTCHPAK™ 9732 polyester film laminate; available from 3M Company). The resulting coating contained 22.0 percent fentanyl, 20.0 percent tetraglycol, and 1.5 percent N, N-dimethyldodecylamine N-oxide. The permeation through human cadaver skin was determined using the test method described above. The results are shown in Table 10 below.

### Example 40

Fentanyl (1.8001 g) was added to methanol (2.0065 g) and mixed until all of the fentanyl was dissolved. To this solution, copolymer (5.5535 g of dried isooctyl acrylate/2-hydroxyethyl acrylate/Elvacite™ 1010/vinyl acetate (57/39/4/10) from Copolymer F above), methyl laurate (2.5003 g), N, N-dimethyldodecylamine N-oxide (0.1511 g), and ethyl acetate (8.0175 g) were added and mixed until a uniform coating formulation was obtained. The coating formulation was knife coated at a wet thickness of 14 mil (355.6 µm) onto a release liner (Daubert 164P silicone coated release liner). The resulting coated liner was oven dried for 4 minutes at 110°F (43°C), for 2 minutes at 185°F (85°C), and for 2 minutes at 225°F (107°C) and then it was laminated onto a backing (SCOTCHPAK™ 9732 polyester film laminate; available from 3M Company). The resulting coating contained 18.0 percent fentanyl, 25.0 percent methyl laurate, and 1.5 percent N, N-dimethyldodecylamine N-oxide. The permeation through human cadaver skin was determined using the test method described above. The results are shown in Table 10 below.

### Example 41

Fentanyl (3.0314 g) was added to methanol (2.9990 g) and mixed until all of the fentanyl was dissolved. To this solution, copolymer (8.7452 g of dried isooctyl acrylate/2-hydroxyethyl acrylate/Elvacite™ 1010/vinyl acetate (57/39/4/10) from Copolymer F above), tetraglycol (3.0040 g), N, N-dimethyldodecylamine N-oxide (0.2250 g), and ethyl acetate (12.0046 g) were added and mixed until a uniform coating formulation was obtained. The coating formulation was knife coated at a wet thickness of 22 mil (558.8 µm) onto a release liner (Daubert 164P silicone coated release liner). The resulting coated liner was dried at room temperature for 4 minutes, and then oven dried for 4 minutes at 110°F (43°C), for 2 minutes at 185°F (85°C), and for 2 minutes at 225°F (107°C) and a portion was laminated onto a backing (SCOTCHPAK™ 9732 polyester film laminate; available from 3M Company). The resulting coating contained 20.2 percent fentanyl, 20.0 percent tetraglycol, and 1.5 percent N, N-dimethyldodecylamine N-oxide. The release liner was removed and the exposed coated surface was laminated to the coated surface of a second section of the coated release liner. The permeation through human cadaver skin was determined using the test method described above. The results are shown in Table 10 below.

### Example 42

Fentanyl (2.5835 g) was added to methanol (2.9991 g) and mixed until all of the fentanyl was dissolved. To this solution, copolymer (8.6686 g of dried isooctyl acrylate/2-hydroxyethyl acrylate/Elvacite™ 1010/vinyl acetate (57/39/4/10) from Copolymer F above), methyl laurate (3.9490 g), and ethyl acetate (12.0020 g) were added and mixed until a uniform coating formulation was obtained. The coating formulation was knife coated at a wet thickness of 22 mil (558.8 µm) onto a release liner (Daubert 164P silicone coated release liner). The resulting coated liner was dried at room temperature for 4 minutes, and then oven dried for 4 minutes at 110°F (43°C), for 2 minutes at 185°F (85°C), and for 2 minutes at 225°F (107°C) and a portion was laminated onto a backing (SCOTCHPAK™ 9732 polyester film laminate; available from 3M Company). The resulting coating contained 17.0 percent fentanyl and 26.0 percent methyl laurate. The release liner was removed and the exposed coated surface was laminated to the coated surface of a second section of the coated release liner. The permeation through human cadaver skin was determined using the test method described above. The results are shown in Table 10 below.

| Table 10 | | | | | | | | | | | | |
|---|---|---|---|---|---|---|---|---|---|---|---|---|
| Human Cadaver Skin Permeation | | | | | | | | | | | | |
| Example Number | Average Cumulative Amount Penetrated (µg/cm²) | | | | | | | | | | | |
| | 4 hr | 8 hr | 12 hr | 24 hr | 32 hr | 48 hr | 60 hr | 72 hr | 96 hr | 120 hr | 144 hr | 168 hr |
| 39 | 28 | 84 | 157 | 423 | 620 | - | - | - | - | - | - | - |
| 40 | 70 | 212 | 390 | 934 | 1237 | - | - | - | - | - | - | - |
| 41 | 2 | 12 | 32 | 122 | 224* | 342 | 447 | 545 | 770 | 979 | 1173 | 1358 |
| 42 | 9 | 32 | 61 | 170 | 277* | 391 | 487 | 576 | 769 | 944 | 1103 | 1246 |

| | | | | | | | | | | | | |
|---|---|---|---|---|---|---|---|---|---|---|---|---|
| *This time point was 36 hours for examples 41 and 42. | | | | | | | | | | | | |

### Example 43

Fentanyl (1.1220 g) was added to methanol (11.9975 g) and mixed until all of the fentanyl was dissolved. To this solution, copolymer (12.8842 g of dried isooctyl acrylate/acrylamide/vinyl acetate (75/5/20) from Copolymer H above), methyl laurate (6.0222 g), and ethyl acetate (48.0729 g) were added and mixed until a uniform coating formulation was obtained. The coating formulation was knife coated at a wet thickness of 24 mil (609.6 µm) onto a release liner (Daubert 164P silicone coated release liner). The resulting coated liner was oven dried for 4 minutes at 110°F (43°C), for 2 minutes at 185°F (85°C), and for 2 minutes at 225°F (107°C) and a portion was laminated onto a backing (SCOTCHPAK™ 9732 polyester film laminate; available from 3M Company). The resulting coating contained 5.6 percent fentanyl and 30.1 percent methyl laurate. The release liner was removed and the exposed coated surface was laminated to the coated surface of a second section of the coated release liner. The permeation through human cadaver skin was determined using the test method described above. The results are shown in Table I 1 below.

### Example 44

Fentanyl (0.5610 g) was added to methanol (5.9945 g) and mixed until all of the fentanyl was dissolved. To this solution, copolymer (6.4317 g of dried isooctyl acrylate/vinyl acetate/Elvacite™ 1010 (56/38/6) from Copolymer G above), methyl laurate (3.0226 g), and ethyl acetate (24.0350 g) were added and mixed until a uniform coating formulation was obtained. The coating formulation was knife coated at a wet thickness of 24 mil (609.6 µm) onto a release liner (Daubert 164P silicone coated release liner). The resulting coated liner was oven dried for 4 minutes at 110°F (43°C), for 2 minutes at 185°F (85°C), and for 2 minutes at 225°F (107°C) and a portion was laminated onto a backing (SCOTCHPAK™ 9732 polyester film laminate; available from 3M Company). The resulting coating contained 5.6 percent fentanyl and 30.2 percent methyl laurate. The release liner was removed and the exposed coated surface was laminated to the coated surface of a second section of the coated release liner. The permeation through human cadaver skin was determined using the test method described above. The results are shown in Table 11 below.

| Table 11 | | | | | | | | | | | |
|---|---|---|---|---|---|---|---|---|---|---|---|
| Human Cadaver Skin Permeation | | | | | | | | | | | |
| Example Number | Average Cumulative Amount Penetrated (µg/cm²) | | | | | | | | | | |
| | 2 hr | 4 hr | 8 hr | 12 hr | 24 hr | 48 hr | 72 hr | 96 hr | 120 hr | 144 hr | 168 hr |
| 43 | 0.1 | 1 | 9 | 21 | 69 | 180 | 290 | 375 | 447 | 501 | 548 |
| 44 | 0.5 | 2 | 11 | 22 | 68 | 180 | 289 | 372 | 438 | 485 | 523 |

### Example 45

Fentanyl (0.2732 g) was added to methanol (2.9986 g) and mixed until all of the fentanyl was dissolved. To this solution, copolymer (3.3097 g of dried isooctyl acrylate/acrylamide/vinyl acetate (75/5/20) from Copolymer H above), methyl laurate (1.4252 g), and ethyl acetate (12.0460 g) were added and mixed until a uniform coating formulation was obtained. The coating formulation was knife coated at a wet thickness of 19 mil (482.6 µm) onto a release liner (Daubert 164P silicone coated release liner). The resulting coated liner was oven dried for 10 minutes at 110°F (43°C) and a portion was laminated onto a backing (SCOTCHPAK™ 9732 polyester film laminate; available from 3M Company). The resulting coating contained 5.5 percent fentanyl and 28.5 percent methyl laurate. The release liner was removed and the exposed coated surface was laminated to the coated surface of a second section of the coated release liner. The permeation through human cadaver skin was determined using the test method described above. The results are shown in Table 12 below.

### Example 46

A fentanyl stock solution was prepared by adding fentanyl (0.7094 g) to methanol (1.7339 g) and mixing until all of the fentanyl was dissolved. Copolymer (3.4998 g of dried isooctyl acrylate/acrylamide/vinyl acetate (75/5/20) from Copolymer H above), methyl laurate (3.0293 g), and ethyl acetate (12.1824 g) were combined and mixed until a uniform formulation was obtained. To this uniform formulation, a portion of the fentanyl, stock solution (0.5471) was added and mixed until a uniform coating formulation was obtained. The coating formulation was knife coated at a wet thickness of 19 mil (482.6 µm) onto a release liner (Daubert 164P silicone coated release liner). The resulting coated liner was oven dried for 10 minutes at 110°F (43°C) and then it was laminated onto a backing (SCOTCHPAK™ 9732 polyester film laminate; available from 3M Company). The resulting coating contained 5.9 percent fentanyl and 28.3 percent methyl laurate. The permeation through human cadaver skin was determined using the test method described above. The results are shown in Table 12 below.

| Table 12 | | | | | | | | | | |
|---|---|---|---|---|---|---|---|---|---|---|
| Human Cadaver Skin Permeation | | | | | | | | | | |
| Example Number | Average Cumulative Amount Penetrated (µg/cm²) | | | | | | | | | |
| | 3 hr | 6 hr | 9 hr | 12 hr | 18 hr | 24 hr | 32 hr | 48 hr | 56 hr | 72 hr |
| 45 | 19 | 45 | 77 | 111 | - | 239 | - | 473 | - | 594 |
| 46 | 14 | 46 | 87 | 125 | 193 | 249 | 303 | 384 | 408 | 436 |

### Example 47

A transdermal coating was prepared following substantially the same procedure as Example 41. The resulting coating contained 20.2 percent fentanyl, 20.0 percent tetraglycol, and 1.5 percent N, N-dimethyldodecylamine N-oxide. Transdermal patches with an active surface area of 20 cm² were converted from the coating. Permeation through human skin was determined by applying one patch each to fourteen healthy human test subjects. Blood sampling was performed at fixed time intervals to determine plasma fentanyl concentrations in the subjects. The results are shown in Table 13 below.

### Example 48

A transdermal coating was prepared following substantially the same procedure as Example 42. The resulting coating contained 17.2 percent fentanyl and 25.0 percent methyl laurate. Transdermal patches with an active surface area of 20 cm² were converted from the coating. Permeation through human skin was determined by applying one patch each to twelve healthy human test subjects. Blood sampling was performed at fixed time intervals to determine plasma fentanyl concentrations in the subjects. The results are shown in Table 13 below.

| Table 13 | | | | | | | | | | | |
|---|---|---|---|---|---|---|---|---|---|---|---|
| Human Skin Permeation | | | | | | | | | | | |
| Example Number | Average Fentanyl Plasma Level (µg/mL) | | | | | | | | | | |
| | 2 hr | 4 hr | 8 hr | 12 hr | 24 hr | 48 hr | 72 hr | 96 hr | 120 hr | 144 hr | 168 hr |
| 47 | - | 0.31 | 1.27 | 1.71 | 3.24 | 3.32 | 3.01 | 2.87 | 2.78 | 2.39 | 2.39 |
| 48 | - | 0.25 | 1.06 | 1.44 | 2.58 | 2.75 | 2.64 | 2.55 | 2.46 | 2.33 | 2.27 |

### Examples 49-54

Fentanyl (10.014 g) was added to methanol (11.64 g) and mixed until all of the fentanyl was dissolved. To this solution, copolymer (33.649 g of dried isooctyl acrylate/2-hydroxyethyl acrylate/Elvacite™ 1010/vinyl acetate (57/3914110) from Copolymer E above), methyl laurate (14.551 g), and ethyl acetate (46.58 g) were added and mixed until a uniform coating formulation was obtained. Portions of the coating formulation were knife coated onto release liner (Daubert 164P silicone coated release liner) to produce reservoir layers with dry coating weights of 10.0 to 12.0 mg/cm². The resulting coated liner was laminated onto a backing (SCOTCHPAK™ 9732 polyester film laminate; available from 3M Company). Portions of the coating formulation were also knife coated onto release liner (Daubert 164P silicone coated release liner) to produce skin contact layers with dry coating weights of 3.0 to 5.0 mg/cm². The resulting coated liner was laminated onto a membrane (ethylene:vinyl acetate membrane with varying percentages of vinyl acetate). In each example, the liner from the reservoir layer was removed and the surface of the membrane opposed to the skin contact layer was laminated to the reservoir layer to prepare a membrane rate controlled device. The resulting coatings contained 17.2 percent fentanyl and 25.0 percent methyl laurate. The reservoir layer coat weight, skin contact layer coat weight, and percentage of vinyl acetate in the membrane for each example is given in Table 14 below. The permeation through human cadaver skin was determined using the test method described above. The results are shown in Table 15 below.

| Table 14 | | | |
|---|---|---|---|
| Example Number | Membrane % vinyl acetate | Reservoir Layer Coat Wt. [g/cm²] | Skin Contact Layer Coat Wt. [g/cm²] |
| 49 | 2.0 | 12.0 | 3.0 |
| 50 | 4.5 | 12.0 | 3.0 |
| 51 | 2.0 | 11.0 | 4.0 |
| 52 | 4.5 | 11.0 | 4.0 |
| 53 | 2.0 | 10.0 | 5.0 |
| 54 | 4.5 | 10.0 | 5.0 |

| Table 15 | | | | | | | | | | | |
|---|---|---|---|---|---|---|---|---|---|---|---|
| Human Cadaver Skin Permeation | | | | | | | | | | | |
| Example Number | Average Cumulative Amount Penetrated (µg/cm²) | | | | | | | | | | |
| | 4 hr | 8 hr | 12 hr | 23 hr | 32 hr | 48 hr | 72 hr | 96 hr | 120 hr | 144 hr | 168 hr |
| 49 | 12 | 34 | 62 | 154 | 231 | 358 | 513 | 664 | 790 | 898 | 994 |
| 50 | 13 | 37 | 68 | 167 | 252 | 391 | 566 | 746 | 889 | 1022 | 1137 |
| 51 | 5 | 22 | 44 | 123 | 195 | 320 | 476 | 633 | 754 | 867 | 969 |
| 52 | 8 | 24 | 47 | 129 | 204 | 335 | 504 | 683 | 825 | 957 | 1074 |
| 53 | 5 | 20 | 42 | 124 | 202 | 339 | 512 | 685 | 819 | 938 | 1044 |
| 54 | 10 | 29 | 53 | 137 | 214 | 353 | 533 | 718 | 863 | 995 | 1112 |

The present invention has been described with reference to several embodiments thereof. The foregoing detailed description and examples have been provided for clarity of understanding only, and no unnecessary limitations are to be understood therefrom. It will be apparent to those skilled in the art that many changes can be made to the described embodiments without departing from the spirit and scope of the invention. Thus, the scope of the invention should not be limited to the exact details of the compositions and structures described herein, but rather by the language of the claims that follow.

## Claims

1. A device for the transdermal delivery of fentanyl comprising a backing and a transdermal drug delivery composition, said transdermal drug delivery composition being adhered to one surface of the backing, wherein said transdennal drug delivery composition comprises
(a) a copolymer comprising
(i) one or more A monomers selected from the group consisting of alkyl acrylates containing 4 to 12 carbon atoms in the alkyl group and alkyl methacrylates containing 4 to 12 carbon atoms in the alkyl group; and
(ii) one or more ethylenically unsaturated B monomers copolymerizable with the A monomer; and
(b) about 8% to about 30% by weight fentanyl based on the total weight of the composition.

2. The device of claim 1 wherein the A monomer is selected from the group consisting of isooctyl acrylate, 2-ethylhexyl acrylate, butyl acrylate, and cyclohexyl acrylate.

3. The device of claim 1 or 2 wherein the B monomer is selected from the group consisting of 2-hydroxyethyl acrylate, 2-hydroxyethyl methacrylate, glyceryl acrylate, N,N-diethylacrylamide, 2-ethoxyethoxyethyl acrylate, 2-ethoxyethyl acrylate, tetrahydrofurfuryl acrylate, acrylic acid, acrylamide, vinyl acetate, N-vinyl pyrrolidone and mixtures thereof.

4. The device of claim 3 wherein the B monomer is 2-hydroxyethyl acrylate.

5. The device of any one of claims 1 to 4 wherein the copolymer further comprises a macromonomer.

6. The device of claim 5 wherein the macromonomer is a functionally terminated polymethylmethacrylate.

7. The device of claim 5 or 6 wherein the copolymer contains from about 1% to about 6% of macromonomer by weight based on the total weight of all monomers in the copolymer.

8. The device of any one of claims 1 to 7 wherein the composition further comprises a delivery enhancing adjuvant.

9. The device of claim 8 wherein the delivery enhancing adjuvant is selected from the group consisting of alkane polyols, fatty acids, fatty acid esters, fatty alcohols, terpenes, C₅-C₁₈ alkyl esters of a carboxylic acid, and mixtures thereof.

10. The device of any one of claims 1 to 9 wherein the concentration of fentanyl in said transdermal drug delivery composition is from about 12% to about 24% by weight.

11. The device of any one of claims 1 to 10 wherein the composition is substantially free of undissolved fentanyl.

12. The device according to any one of claims 1 to 11 wherein the device is configured to deliver fentanyl to a mammal in an amount of about 0.5 to about 5.0 mg/day thereby causing the serum concentration of fentanyl in the mammal to be about 0.2 to about 10 ng/mL for a period of time from about 4 to about 14 days.

13. A device for the transdermal delivery of fentanyl comprising:
(a) a drug reservoir layer comprising the composition as defined in any of claims 1 to 12;
(b) a rate controlling membrane adhered to one surface of the drug reservoir layer; and
(c) a skin contacting pressure sensitive adhesive layer adhered to the surface of the membrane that is opposed to the surface of the membrane in contact with the reservoir layer.

14. Use of a transdermal drug delivery composition for the preparation of a device for the transdermal delivery of fentanyl as defined in any one of claims 1 to 13 wherein the device is for providing analgesia or for the treatment of a condition capable of treatment by fentanyl.

15. A device for the transdermal delivery of fentanyl as defined in any one of claims 1 to 13 wherein the device for use in providing analgesia or for use in the treatment of a condition capable of treatment by fentanyl.
